# EUROPEAN PATENT SPECIFICATION

(11) **EP 0 276 262 B1**
(45) Date of publication and mention of the grant of the patent: **23.10.1996**
(21) Application number: 87904811.4
(22) Date of filing: 17.07.1987
(51) Int. Cl.: C07K 14/445, C12N 1/20, C12N 5/00, C12N 15/00, C12P 21/02, A61K 39/015

(54) **MEROZOITE SURFACE ANTIGEN OF PLASMODIUM FALCIPARUM**
MEROZOIT-OBERFLÄCHEN-PLASMODIUM-FALCIPARUM-ANTIGEN
ANTIGENE DE PLASMODIUM FALCIPARUM APPARAISSANT A LA SURFACE DE MEROZOITES

(30) Priority: 17.07.1986 AU 6977/86; 24.03.1987 AU 1047/87; 24.03.1987 AU 1048/87
(43) Date of publication of application: 03.08.1988
(73) Proprietor: SARAMANE PTY. LTD., Parkville, VIC 3052 (AU)
(72) Inventor: EPPING, Ronald, Jof, Herston, QLD 4006 (AU); RAMASAMY, Ranjan, Chappel Hill, QLD 4069 (AU); SMYTHE, Jason, Arthur, Edithvale, VIC 3196 (AU); ANDERS, Robin, Fredric, North Melbourne, VIC 3051 (AU); COPPEL, Ross, Leon, Armadale, VIC 3143 (AU); GEYSEN, Hendrik, Mario, Knoxfield, VIC 3180 (AU); SAUL, Allan, James, The Gap, QLD 4061 (AU)
(74) Representative: Dzieglewska, Hanna Eva
(86) International application number: AU8700227
(87) International publication number: WO8800595

(56) References cited:
- AU-A- 5 540 886
- AU-B- 0 561 529
- LYON, J.A. et al. The Journal of Immunology, Volume 38, No. 3, issued 1987 February (WILLIAMS and WILKINS Co., Baltimore, U.S.A.), 'Monoclonal Antibody Characterization of the 195-Kilodalton Major Surface Glycoprotein of Plasmodium Flaciparum Malaria Schizonts and Merozoites : Identification of Additional Processed Products and Serotype- Resticted Repetitive Epitope , see pages 895-901
- HALDER, K. et al. The Journal of Biological Chemistry, Volume 260, No. 8, issued 1985 April (American Society of Biological Chemists, Inc., Bethesda, USA), 'Acylation of a Plasmodium Falciparum Merozoite Surface Antigen via Sn-1, 2-Diacyl Glycerol', see pages 4969 to 4974
- LYON, J.A. and J.D. HAYNES, The Journal of Immunology, Volume 136, No. 6, issued 1986 March (Williams and Wilkins Co., Baltimore, USA), 'Plasmodium Falciparum Antigens Synthesized by Schizonts and Stabilized at the Merozoite Surface when Schizonts Mature in the Presence of Protease Inhibitors', see pages 2245 to 2251
- LYON, J.A. et al. The Journal of Immunology, Volume 136, No. 6, issued 1986 March (WILLIAMS and WILKINS Co., Baltimore, USA), 'Plasmodium Falciparum Antigens Synthesized by Schizonts and Stabilized at the Merozoite Surface by Antibodies when Schizonts Mature in the Presence of Growth Inhibitory Immune Serum', see pages 2252 to 2258
- HORWARD, R.F. and R.T. REESE. Molecular and Biochemical Parasitology, Volume 10, issued 1984 (Elsevier/North-Holland, New York, USA), 'Synthesis of Merozoite Proteins and Glycoproteins During the Schizogony of Plasmodium Falciparum', see pages 319 to 334
- ARDESHIR, F. et al. Proceedings of the National Academy of Sciences (USA), Volume 82, issued 1985 April, 'Expression of Plasmodium Falciparum Surface Antigens in Escherichia Coli', see pages 2518 to 2522
- HOLDER, A.A. et al. Nature, Volume 317, issued 1985 September (USA), 'Primary Structure of the Precursor to the Major Surface Antigens of Plasmodium Falciparum Merozoites', see pages 270 to 273
- J Immunol 134 (1985) 3439-3444
- Molec Biochem Parasitol 34 (1989) 79-86
- Molec Biochem Parasitol 10 (1984) 319-334

## Description

This invention relates to the identification of antigens of the asexual blood stages of Plasmodium falciparum, which are capable of generating antibodies which are able to inhibit the growth of the parasite, and to the use of these antigens and antibodies in immunization, diagnostic and treatment methods.

Merozoite surface antigens are of particular interest in the study of malarial immunology and the mechanism of host protection to parasites due to their accessibility to host immune mechanisms and because of their likely involvement in the erythrocytic invasion process. Immunity against erythrocytic stages of Plasmodium infections has been demonstrated by simian vaccination studies using merozoite preparations of P.knowlesi (1) and P.falciparum (2,3). Antigens identified by ¹²⁵I-surface labelling studies (4,5) are likely to reside on the merozoite surface membrane whereas the localization of those antigens which are immuno-precipitated with immune serum (6,8) is more tenuous. One high Mr antigen of Plasmodium falciparum (9) which is synthesised predominantly in schizonts and processed to generate proteins of Mr 88, 42 and 18 kDa (10,11) upon schizont maturation is the precursor to the major merozoite surface antigen (PMMSA). This group of antigens provide a degree of immunity to malaria in saimiri monkeys (12). They have been described in several Plasmodium falciparum strains (9, 13-15) and Plasmodial species (16,17). Several proteins and glycoproteins of Mr 35-45 kDa and 50-60 kDa from Plasmodium falciparum have been detected using ¹²⁵I-surface labelling of mature schizont-stage parasites (4,18) however, a precursor/product relationship with high Mr antigens has not been ascribed to these.

Haldar et al., in J. Biol. Chem. 260, 4969-4974, (1985) and Lyon et al., in J. Immunol. 138, 895-901, (1987) describe merozoite surface antigens derived from a 195 kDa glycoprotein, which is PMMSA.

Stanley et al., in J. Immunol 134, 3439-3444, (1985) describe a merozoite surface glycoprotein of molecular weight 56 kDa isolated from the FVO and Geneva isolates of P.falciparum.

Howard and Reese in Mol. Biochem. Parasitol; 10, 319-334, (1984) describe five glycoproteins of molecular weight 185, 88, 56, 46 and 34 kDa expressed on merozoites of the FVO isolate of P.falciparum.

One method for the identification of antigens of the asexual blood stages of the Plasmodium falciparum parasite which can be used to generate antibodies able to inhibit the growth of said parasite comprises:
(a) growing Plasmodium falciparum isolates in vitro thereby producing erythrocytes infected with the Plasmodium falciparum parasite;
(b) purifying these erythrocytes;
(c) immunising mice with the purified erythrocytes;
(d) fusing spleen cells from the immunised mice with a mouse myeloma cell line to produce hybridomas;
(e) testing the monoclonal antibodies produce by the hybridomas to identify those hybridoma lines which secrete monoclonal antibodies against the Plasmodium falciparum parasite;
(f) characterising the monoclonal antiparasite antibodies;
(g) preparing mouse ascites fluid by injecting mice intraperitoneally with hybridoma cells secreting monoclonal antiparasite antibodies;
(h) extracting and purifying monoclonal antibodies from these ascites fluids;
(i) adding the purified antibodies to Plasmodium falciparum cultures in vitro and subsequently examining the cultures for a decrease in the multiplication rate of the parasites;
(j) characterising the antigen recognised by each inhibitory monoclonal antibody;
(k) identifying the cellular distribution of the antigen and the stage of parasite maturation during which it is present.

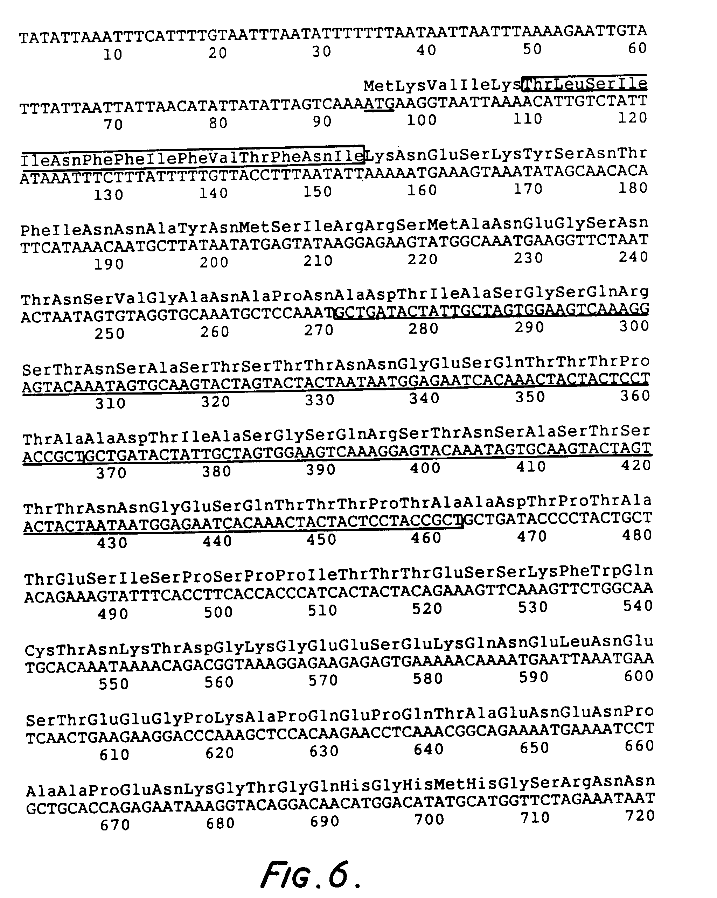

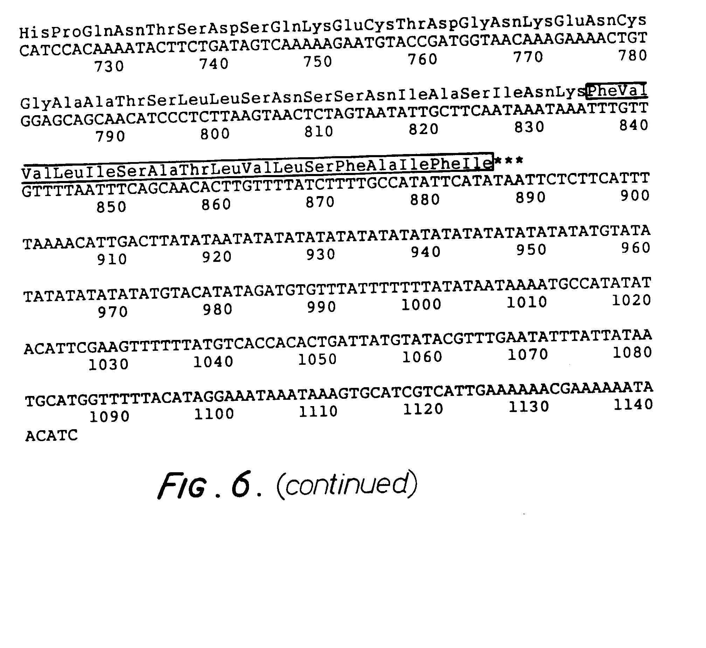

According to one aspect of the present invention, there is provided an isolated merozoite surface antigen of the asexual blood stages of Plasmodium falciparum, other than the FVO and Geneva isolates of P.falciparum, which is characterised by:
- (i): having an apparent molecular weight in the range of approximately 41 kDa to 53 kDa by reducing SDS-PAGE;
- (ii): being a glycoprotein incorporating myristic acid;
- (iii): being present firstly as a diffuse cytoplasmic localisation and in mature schizonts on the surface membrane of merozoites;
- (iv): being recognised by monoclonal antibodies against the asexual blood stages of P.falciparum which inhibit parasite growth in vitro; and
- (v): when isolated from strain FCQ-27/PNG, having the amino acid sequence set out in Figure 6;
and which does not derive from the 195 kDA precursor to the major merozoite surface antigen (PMMSA); or an antigenic fragment of said antigen that elicits a specific immune response in a host.

In a preferred embodiment, the antigen is characterised by inclusion in the amino acid sequence thereof of the sequence Ser-Thr-Asn-Ser (STNS) or the sequence Ser-Asn-Thr-Asn-Ser-Val (SNTNSV).

Preferably, the antigen is antigen QF122 (also called GYMSSA) described in detail herein, having the amino acid sequence set out in Figure 6, or an antigenic fragment thereof, that elicits a specific immune response in a host.

The size of the antigen of the present invention on a series of polyacrylamide gels has been ascertained using monoclonal antibodies 8G10/48, 9E3/48 and 8F6/49 described herein, as well as with certain affinity-purified naturally occurring human antibodies. All antibodies give identical bands on Western blotting, and the principal band recognised migrates as a diffuse band with apparent sizes of 53 kDa, 51 kDa, 44 kDa and 41 kDa on 7.5%, 10%, 12.5% and 15% acrylamide gels respectively when compared with the mobility of standard proteins.

Following investigation of the deduced amino acid sequence of the antigen of the present invention, and the putative epitope thereof, by a scanning technique, it has been determined that certain sequences of the antigen bind with inhibitory monoclonal antibodies which recognise the antigen of the invention.

Thus, a hybrid cell has been prepared which produces an antibody which is specific for an antigen of the present invention as well as antibodies produced by such a hybrid cell line.

These include for example the monoclonal antibodies 8G10/48, 9E3/48 or 8F6/49 described in detail herein. Monoclonal antibody 8G10/48 is produced by the hybrid cell line deposited at the European Collection of Animal Cell Cultures, Porton Down, Salisbury, England, on July 10, 1987 under No. 87071010.

In a preferred aspect, the present invention also relates to an antigen of Plasmodium falciparum as hereinbefore described, which is recognised by the monoclonal antibody 8G10/48.

The monoclonal antibodies which have prepared, which are specific for the antigens of the present invention, are characterised in that they either prevent release of merozoites into the blood stream of an infected individual, or prevent these merozoites from invading the red blood cells of such an individual. such antibodies may be used for the preparation of a vaccine for passively immunising a host against Plasmodium falciparum.

The invention also provides use of an antigen according to the present invention, or an antigenic fragment thereof that elicits a specific immune response in a host, for the preparation of a composition for actively immunising a host against Plasmodium falciparum.

The invention provides a vaccine comprising an antigen of the present invention, or an antigenic fragment thereof that elicits a specific immune response in a host, a pharmaceutically acceptable carrier or diluent, and optionally an adjuvant.

Antibodies such as those defined above may be used as a reagent for purification of Plasmodium falciparum antigens or as a diagnostic reagent for detection of Plasmodium falciparum or antigens derived therefrom. .

As a result of applying the general techniques described above, a range of monoclonal antibodies have been identified as able to inhibit the growth of malaria in vitro, and these monoclonal antibodies have been investigated in greater detail as outlined above.

Monoclonal antibodies were purified by affinity chromatography on Protein A Sepharose (Pharmacia) and were tested for inhibition of invasion using a similar method to that described in Schofield (19). Briefly, purified monoclonal antibodies in 1640 medium were added to synchronised parasites at ring stage and incubation of the parasites continued to allow reinvasion. [³⁵S] Methionine (3-4µCi) or [³H]-hypoxanthine (3-4µCi) was added to each well and growth of the parasite allowed to proceed to trophozoite stage. The samples were harvested, protein precipitated with ice cold 10% trichloro-acetic acid onto glass fibre filters, the filters were washed and the radioactivity remaining was measured in a scintillation counter. The class and subclass was determined by immunodiffusion in agar using rabbit antisera specific for mouse IgM, IgGl, IgG2a, IgG2b and Ig3.

### EXAMPLE 1

### Material and Methods

### Parasites

Parasite line FCQ-27/PNG was grown in culture by a modification (21) of the method of Trager and Jensen (22). The parasites were grown in synchronous culture using multiple sorbitol treatments as described (23).

### Antibodies

Murine hybridomas were produced by the method of Galfre et.al. (24) as described (25).

The immunisation regime and fusion were performed by immunising a mouse with 10⁷ red cells infected with mature schizonts of the PNG isolate FCQ-27/PNG. These cells were purified from culture by the method of Saul et.al. (41). The cells were injected intraperitoneally with 0.1 ml Freund's complete adjuvant. The mice were boosted 4 weeks later by an intravenous injection of 5 x 10⁶ schizont infected red cells in saline. A similar boost was given after a further 2 weeks. Four days after the last injection, the spleen cells were harvested and fused.

Monoclonal antibodies 8G10/48 and 9E3/48 belong to the IgG_{2b} subclass as determined by Ouchterlony immunodiffusion in agarose. Monoclonal antibody 8F6/49 is an IgG₃ antibody. Antibodies were labelled with ¹²⁵I using the Iodogen procedure of Fraker and Speck (26).

### Immunofluorescence assay

Thin films of schizont-stage parasite cultures with a parasitemia > 5% were fixed for 20min in acetone at -20°C and air-dried. Slides were incubated overnight at 4°C with aliquots of hybridoma supernatants at high humidity and washed twice for 15min with PBS. Fifty microlitres of fluorescein-conjugated, affinity-purified goat anti-mouse IgG (1 µg ml⁻¹, Kirkegaard and Perry Lab.Inc., Maryland, USA) in PBS containing 5% (w/v) low-fat milk powder were applied as the second antibody. Following 2h incubation at 20°C films again were washed twice in PBS for 15min and blotted dry. Slides were mounted in 90% (v/v) glycerol containing 0.2M Tris, 50 µg ml⁻¹ N-propyl gallate and examined using an Olympus BH2 U.V. fluorescence microscope.

In an alternative procedure, thin smears were made from schizont stage parasites isolated by gelatine sedimentation and the cells fixed in acetone: methanol 9:1 at -20°C for 30min. The parasites were reacted with Mab 8F6/49 (supernatants of hybridoma cultures used undiluted) at 4°C for 16h, washed in 0.01M phosphate buffered saline, pH 7.2 (PBS), and then treated with fluorescein conjugated goat antibodies to mouse immunoglobulins for 3h at 37°C. The washed cells were then viewed under U.V. illumination in an Olympus microscope with appropriate filters.

### Immunoelectron microscopy

Parasitized erythrocytes were harvested from synchronous cultures in the presence of leupeptin and antipain (10 µg ml⁻¹) to reduce invasion of erythrocytes and promote the accumulation of meorzoites clustered around haemazoin pigment granules (27). Parasitised cells were incubated for 30min at 20°C with monoclonal antibody (50 µg ml⁻¹) in 100mM Na₂HPO₄ (pH7.4) with intermittent agitation. Antibodies recognising intra-parasite antigens were used as controls. The cells were washed three times in phosphate buffer and incubated for 30min with goat anti-mouse colloidal gold (Janssen Life Sciences, 1/20 dilution in 100mM Na₂HPO₄) with constant agitation. Cells then were rinsed in phosphate buffer prior to fixation for one hour in 100mM sodium cacodylate/HCl buffer (pH7.4) containing 120mM sucrose and 3% (v/v) glutaraldehyde. Fixed cells were washed in phosphate buffer, post fixed in 2% (w/v) osmium tetroxide for lh and 2% (w/v) uranyl acetate for 30min. Finally, the fixed cells were dehydrated in a series of alcohols and embedded in Spurrs resin for 24h at 70°C. Ultrathin sections (0.06µ) were cut using a diamond knife and mounted on nickel grids. Sections were contrasted using uranyl acetate and lead citrate and examined using a Philips EM 400 electron microscope.

### Inhibition of Parasite growth in vitro

Protein A affinity-purified antibody was added to highly-synchronised cultures of FCQ-27/PNG in 96-well plates. The wells contained 45µl culture medium, 10% (v/v) pooled human plasma (0.01-1.0 mg ml⁻¹) with a 5% haematocrit and 1-2% schizont parasitemia and 5µl of test antibody. The culture medium contained 11.1mM glucose, 40.3mM TES, 27.6mM NaHCO₃ and RPMI 1640. (Gibco Labs, Life Technologies Inc., Grand Island, N.Y., U.S.A.). Control wells were identical in all respects except that they contained either protein A affinity-purified normal mouse IgG or medium alone. Experiments were performed in quadruplicate. The cultures were incubated overnight at 37°C. Following merozoite release and subsequent re-invasion, 100µl of culture medium were added supplemented with 30 µCi ml⁻¹ [³H]hypoxanthine (Amersham). The parasites were allowed to develop for 40h to the mature schizont stage and harvested by pipetting the well contents onto Whatman glass fibre discs which then were washed in trichloroacetic acid and ethanol. The dry discs were immersed in 3ml toluene scintillant and counted in a Packard scintillation counter.

### Western blotting

Mature schizont stages of the FCQ-27/PNG strain were extracted with 1% (v/v) Triton X-100 in 150mM NaCl, 50mM Tris (pH7.4), 5mM EDTA and the following protease inhibitors: 5-10 µg ml⁻¹ chymostatin, antipain, leupeptin, N-tosyl-L-phenylalanine chloromethyl ketone (TPCK) and 34 µg ml⁻¹ phenylmethylsulfonyl fluoride (PMSF). Detergent-soluble extracts were boiled in Laemmli sample buffer (28) containing 5% (v/v) β-mercaptoethanol and aliquots were separated onto a discontinuous SDS-slab polyacrylamide gel (3% stacking gel, 7.5% separating gel) with concentrations ranging from 7.5% to 15% acrylamide in particular experiments. The gels were Western blotted onto nitrocellulose (29). Reactive antigens were detected by the binding of murine monoclonal antibody from hybridoma supernatants (1-10 µg ml⁻¹) and [¹²⁵I] goat anti-mouse antibody (0.25 µg ml⁻¹, 1.3 µCi ml⁻¹) followed by autoradiography. [¹⁴C]-labelled Mr standards were from Amersham.

### Immunoprecipitation.

The oligosaccharide chains of parasite glycoproteins were labelled with [6,³H] glucosamine (Radiochemical Centre, Amersham, UK) utilising the late stages of the FC27-D10 clone essentially as described (31). Briefly, 50µl of parasitised cells were cultured overnight in 10ml medium containing lmCi [6, ³H] glucosamine. Radioactive labelling of trophozoites with [³H] myristic acid was performed according to Haldar, et.al. (31a). The parasites then were washed and lysed in 1ml of radioimmunoprecipitation buffer. 200µl aliquots of the lysate were reacted with 50µl of monoclonal antibody (1 mg ml⁻¹) conjugated to Protein A-Sepharose beads. The beads were washed, extracted in Laemmli sample buffer (28) and analysed by SDS-PAGE followed by fluorography.

### LEGENDS TO FIGURES.

- Figure 1:: Indirect immunofluorescence staining pattern produced on acetone-fixed films of Plasmodium falciparum schizonts with monoclonal antibody 8G10/48. Surface fluorescence is localised to the merozoite membrane within the mature schizont. The same fluorescence pattern is observed using monoclonal antibody 9E3/48.
- Figure 2:: Immunoelectromicrograph of merozoites incubated with monoclonal antibody 8G10/48 and colloidal gold:goat anti-mouse Ig. Aggregates of gold particles (15nm diameter) are distributed over the entire surface of the merozoite. Bar = 0.5µ.
- Figure 3a:: Western blot of ring (R), trophozoite (T) and schizont (S) stage parasite proteins probed with Mab 8F6/49. The migration positions of molecular weight markers are indicated.
- Figure 3b:: Fluorograph of immune-precipitates of [³H] glucosamine (a,c) and [³H] myristic acid (b,d) labelled parasite proteins; a,b-control immune precipitates without Mab; c,d-immune precipitates with Mab 8F6/49. The migration positions of molecular weight markers are indicated.

### RESULTS

### Immunofluorescence and immunoelectron microscopy

Murine monoclonal antibodies wre produced against the FCQ-27/PNG strain of Plasmodium falciparum using standard hybridoma technology. Hybridoma supernatants were characterised for cytological epitope localisation using an indirect immunofluorescence assay and immunoelectron microscopy and for functional activity using an in vitro erythrocyte invasion assay.

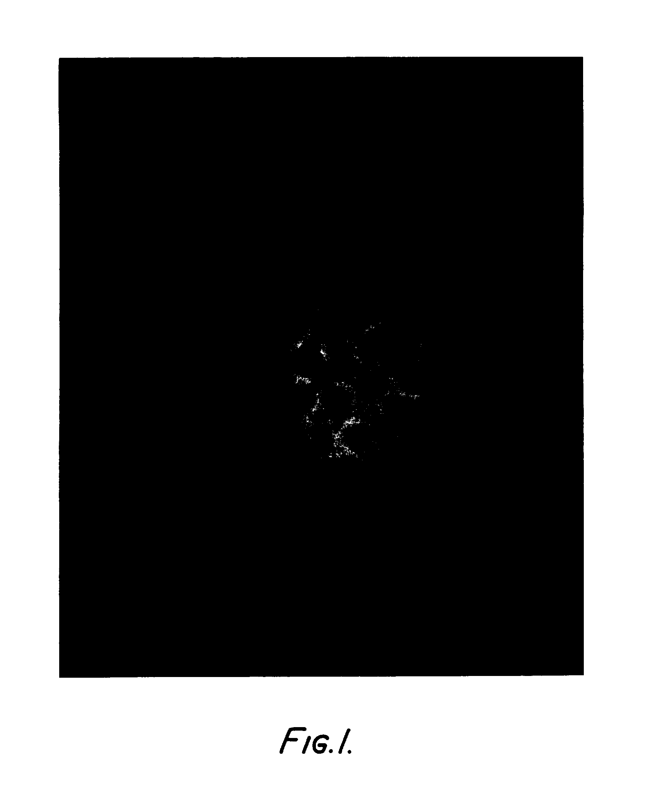

Determinants present on the merozoite surface during schizogony were recognised by monoclonal antibodies 8G10/48, 9E3/48 and 8F6/49. A characteristic multi-spherical fluorescence pattern was observed using immunofluorescence in cultures collected prior to rupture of the mature schizont (Fig.1). Antibody-specific fluorescence was observed during all erythrocytic parasite stages but becomes localised to the membrane surrounding merozoites in mature schizonts. Examination of thin films of several laboratory strains of P.falciparum using immunofluorescence and monoclonal antibodies 8G10/48 and 9E3/48 revealed similar surface fluorescence in all of 4 isolates tested (Table 1).

**TABLE 1.**

| | | | |
|---|---|---|---|
| Indirect immunofluorescence assay results for the binding of monoclonal antibodies to thin films of different isolates of Plasmodium falciparum. Results were scored from "-" to "++++" based upon the number of parasitised cells observed which displayed the surface fluorescence depicted in Fig.1. | | | |

| Parasite isolate | Origin | hybridoma | |
|---|---|---|---|
| | | 8G10/48 | 9E3/48 |
| FC-1 | Papua New Guinea | + | - |
| FCQ-27/PNG | Papua New Guinea | ++++ | ++++ |
| FCR-3K+ | The Gambia | ++ | + |
| K1 | Thailand | +++ | +++ |

Mab 8F6/49 gave a bright fluorescence staining of schizont stage parasites, producing a grape like pattern characteristic of reaction with antigens associated with the merozoite surface. The membranes of newly released merozoites present in the preparation were also stained. Similar staining was observed on the K1 isolate of P.falciparum from Thailand. In contrast, no reaction was observed with the FCR (Gambia) strain.

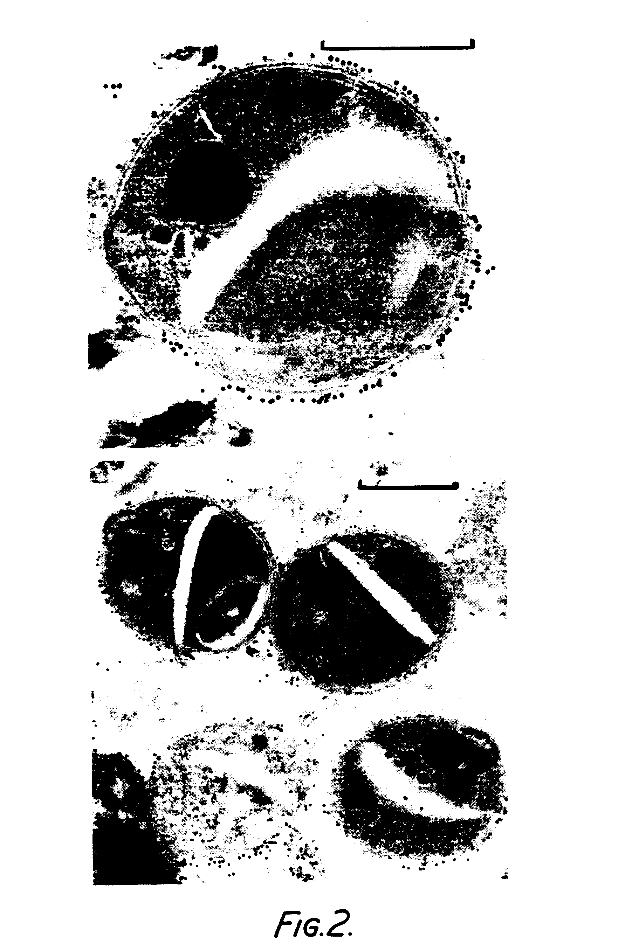

Improved resolution of antibody recognition sites on the merozoite surface was achieved by immunoelectron microscopy of extracellular merozoites utilizing colloidal gold. Dense gold labelling was evident on the merozoite surface membranes, indictive of the relative abundance of this antigen (Fig.2). In addition, gold labelling was seen on extraneous membrane fragments which were present in the preparation. This technique necessitates the careful application of appropriate controls in order to detect the presence of non-specific antibody binding. An absence of labelling was observed in parallel experiments using either a primary monoclonal antibody which recognises an intramerozoite-localised antigen, and goat anti-mouse colloidal gold.

### P.falciparum inhibition studies

Monoclonal antibodies 8G10/48 and 9E3/48 inhibit the growth of Plasmodium falciparum as measured by the uptake of [³⁵S] methionine and [³H] hypoxanthine by highly-synchronised cultures of FCQ-27/PNG (Table 2).

**TABLE 2**

| | | | | | | |
|---|---|---|---|---|---|---|
| Inhibition by monoclonal antibodies of Plasmodium falciparum growth. The effect of monoclonal antibodies 8G10/48 and 9E3/48 on erythrocyte re-invasion by merozoites and parasite replication in vitro was assessed in highly- synchronised cultures of Plasmodium falciparum over a 30hr period commencing at the late schizont stage. Radiolabelled precursors were added at the early ring stage and incorporation was measured at the subsequent schizont stage. Results (mean ± S.E.M.) are shown for six experiments using (a) [³⁵S] methionine or (b) [³H] hypoxanthine. | | | | | | |

| Expt. | Ab. | Ab. Concn. mg ml⁻¹ | n | cpmx10⁻³ | | % inhibn of Control |
|---|---|---|---|---|---|---|
| | | | | Control (Ab. absent) | Test (Ab. present) | |
| 1a | 8G10/48 | 0.112 | 3 | 108±6 | 81±5 | 24 |
| 2b | 8G10/48 | 0.112 | 4 | 926±18 | 689±8 | 26 |
| 3b | 9E3/48 | 0.121 | 4 | 244±11 | 10±1 | 96 |
| 4b | 9E3/48 | 0.121 | 3 | 376±9 | 44±2 | 88 |
| 5b | 9E3/48 | 0.002 | 4 | 348±11 | 249±5 | 28 |
| 6b | 9E3/48 | 0.003 | 4 | 599±8 | 440±8 | 27 |

A figure of 20% inhibition of labelled amino acid incorporation in the assay correlates with a substantial curtailment of parasite development as evidenced by the reduction in the number of ring-infected erythrocytes determined using light microscopy. At an antibody concentration of 112 µg ml⁻¹ monoclonal antibody 8G10/48 gave rise to a 25% inhibition of control rates of amino acid incorporation whilst antibody 9E3/48 produced similar inhibition at 2-3 µl⁻¹. Inhibition levels as high as 96% could be demonstrated in the presence of 121 µg ml⁻¹ 9E3/48. Incorporation of labelled amino acids in these experiments using a number of alternative monoclonal antibodies parallels that observed for control cultures with no added antibody. Western blotting and immunoprecipitation.

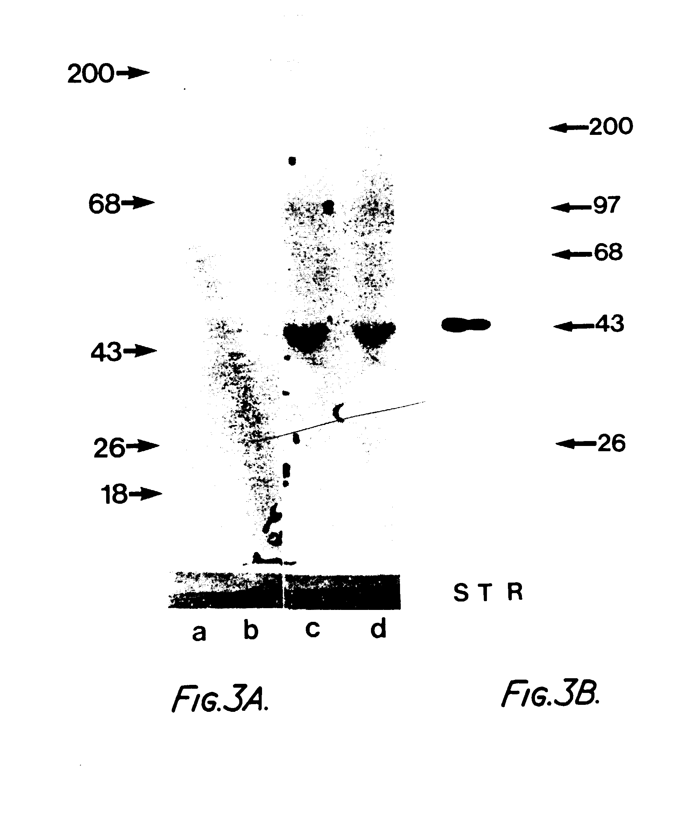

When Western blots of equivalent numbers of mid-ring stage parasites, trophozoites and schizonts, separated on 11% gels, were probed with Mab 8F6/49, a band corresponding to a protein of molecular weight 45,000 Da was detected in the late stage parasites but not in the rings (Fig.3a). The size of the antigen on a series of polyacrylamide gels was ascertained using Mabs 8G10/48, 9E3/48 and 8F6/49 as well as with naturally occurring human antibodies affinity purified as described in Example 2. All antibodies gave identical bands on Western blotting, with the principal band recognised migrating as a diffuse band with apparent sizes of 53 kDa, 51 kDa, 44 kDa and 41 kDa on 7.5%, 10%, 12.5% and 15% acrylamide gels respectively when compared with the mobility of standard proteins (myosin - 200 kDa; phosphorylase b - 92.5 kDa; ovalbumin - 46 kDa; carbonic anhydrase - 30 kDa). The apparent mobility is dependent on the exact gel conditions. These properties are a characteristic of glycoproteins. Immunoprecipitation of [6,³H]glucosamine and [³H] myristic acid labelled parasites revealed the incorporation of both glucosamine and myristic acid into the antigen (Fig.3b).

### EXAMPLE 2

In this example, selective partitioning of integral membrane proteins into the detergent phase upon temperature-dependent phase separation of aqueous solutions of the detergent Triton X-114, has been used to enrich for putative integral membrane proteins of P.falciparum. Human antibodies obtained from patients from Papua New Guinea were then affinity-purified on these antigens after they had been blotted onto nitrocellulose. The purified human antibodies were used to identify recombinant clones of Escherichia coli expressing polypeptide fragments corresponding to these antigens.

### Materials and Methods

### Parasites.

The origin of P.falciparum isolate FCQ27/PNG (FC27) used in these studies has been described elsewhere (42). Parasites synchronized by sorbitol treatment were cultured at 0.25% haematocrit with parasitaemias ranging from 2-7%. All samples were washed free of medium by centrifugation and substitution with human tonicity phosphate buffered saline (HTPBS). Cells were then pelleted, and 1ml packed cell aliquots were snap frozen and stored at -70°C until further processing.

### Triton X-114 solubilization and phase separation.

Triton X-114 solubilization and separation of hydrophobic, hydrophilic and insoluble fractions was performed essentially as described by Bordier (43) with the following modifications. Triton X-114 (purchased from Flulka Ag., Switzerland) was precondensed in human tonicity phosphate buffered saline (HTPBS). A lml aliquot of prepacked parasitized cells was solubilized in 15ml of 0.5% Triton X-114 for 90min on ice, with mild vortexing at 10min intervals. A lml sample of the total material was removed and snap frozen. The remaining 15ml were then centrifuged at 10,000xg for 15min at 4°C to remove insoluble material. The supernatent was removed and this step repeated. The sedimented material (insoluble pellet) was then washed a further three times in 0.5% Triton X-114 before being snap frozen in lml of HTPBS. The detergent-soluble material was then carefully layered over a 10ml sucrose cushion of cold 6% sucrose, 0.06% Triton X-114, placed in a 37°C for 5min waterbath and then centrifuged at 500xg for 5min at 37°C. After centrifugation the 15ml detergent-depleted upper layer was collected and chilled on ice. The 10ml sucrose cushion was discarded and the detergentenriched pellet (1-2ml) was resuspended on ice with 10ml of cold HTPBS. The resuspended detergent enriched phase was again layered over a sucrose cushion, brought to 37° for 5min and repelleted by centrifugation. After this second precipitation, the detergent enriched pellet was resuspended to 5ml in HTPBS and snap frozen. The detergent-depleted upper layer from the sucrose cushion separation was further depleted of hydrophobic proteins by adding lml of 11.4% Triton X-114 on ice, vortexing into solution, warming to 37°C for 5min, then centrifuging and discarding the Triton X-114 pellet. This cycle was repeated three times. The remaining detergent-depleted aqueous solution (aqueous phase) was then snap frozen. All samples were stored at -70°C until analysis.

### Electrophoresis and immunoblotting.

Samples for sodium dodecyl sulphate polyacrylamide gel electrophoresis (SDS-PAGE) were processed under reducing conditions and electrophoresced on 10% slab gels. Gels to be analysed for protein were stained with Coomassie brilliant blue. Samples to be analysed by immunoblotting were fractionated on 10% slab gels and electrophoretically transferred to nitrocellulose sheets. After transfer the nitrocellulose was blocked with 5% (W/V) skim milk powder in HTPBS (Blotto) and probed with sera or affinity purified human antibodies diluted appropriately in Blotto. Bound antibody was detected by probing with ¹²⁵I-protein A followed by autoradiography. Protein A (Pharmacia Fine Chemicals, Uppsala, Sweden) was iodinated by the chloramine T method to a specific activity of 40µCi/µg⁻¹.

### Affinity purification of polyclonal monospecific human antibodies.

Antibodies were affinity purified from human serum or plasma on Triton X-114 soluble antigens electrophoretically transferred to nitrocellulose as described previously (44) and modified in this instance as follows. Reduced samples of Triton X-114 extracted membrane antigens were electrophoretically separated and transferred to nitrocellulose. Radioactive ¹⁴C high molecular weight markers (Amersham) were used to determine the region of interest. Strips of nitrocellulose corresponding to regions of interest were incubated for 8hrs at 4°C with sera or plasma from individuals exposed to malaria. Sera was removed and the strips washed vigorously over 2 hours with 6 changes of Blotto, three changes of HTPBS, followed by a 15 minute wash in borate buffer (0.1 M glycine, 0.15 M sodium chloride, pH 2.6) for 10 minutes. Eluted antibodies were immediately neutralized with 2 M Tris-HCl pH 8.0 and stored at 4°C with 0.05% sodium azide. The affinity-purified antibodies were diluted 1:2 in Blotto to probe immunoblots and λAmp3 cDNA library filters, or concentrated using a Centricon microconcentrator (Amicon) for immunofluorescence assays. Antibodies were also eluted directly from immunopositive λAmp3 clones grown as lawns on nitrocellulose. Filters with lysed lawns were pre-eluted with borate and glycine buffers, incubated with serea, and the monospecific antibodies eluted as above. This second method of affinity purification required the removal of anti E.coli antibodies which was achieved with sonicates and lawns of control λAmp3 clones.

### Identification of cDNA clones expressing relevant polypeptide antigens.

Details of the FCQ27/PNG isolate cDNA library, its amplification in λAmp3 and lysogenic expression E.coli have been described (33). Detection of antigen-expressing clones by in situ colony immunoassay with human antibodies has also been described (33). Positive clones were grown and induced in liquid culture and extracted with sample buffer for electrophoresis as described (45). Samples were electrophoresced on 10% SDS-PAGE slab gels and either stained for protein or immunoblotted and analysed with affinity purified antibodies, to detect the presence or absence of stable fusion polypeptides with β-galactosidase.

### Antibody depletion.

One ml cultures of immunopositive clones were induced, pelleted by centrifugation, and the liquid medium removed. Pellets were frozen and thawed three times and the lysed cells resuspended in lml HTPBS. A 20µl aliquot of PBG sera was added and incubated with the lysate for 3hrs at 4°C. The cellular debris was then spun down at 10,000xg and the supernatant used to probe Triton X-114 extracted and immunoblotted parasite membrane protein antigens in order to determine the degree of antibody depletion attributable to the induced fusion polypeptide.

### Subcloning of immunopositive λAmp3 clones.

λAmp3 phage were isolated from immunopositive clones and the cDNA insert extracted by EcoRI digestion as described. The purified insert was subcloned into M13 vectors for single stranded sequence determined by the dideoxy method of Sanger (36,46) and into pBTA224 (a modification of PUR-290) kindly provided by Gary Coburn (Biotechnology Australia Pty.Ltd.). The plasmid vector pBTA224 is an expressing vector in E.coli cells, and clones were rescreened for immunoreactivity by colony immunoassay.

### Indirect immunofluorescence.

Thin blood films of parasitized erythrocytes from asynchronous cultures of P.falciparum were air dried and fixed in 100% acetone at -20°C for 20 minutes. Slides were incubated with concentrated affinity-purified monospecific human antibodies, with fluorescein-conjugated sheep antihuman Ig antiserum as second antibody. Parasite nuclei were counterstained with ethidium bromide.

### LEGENDS TO FIGURES

- Figure 4:: Triton X-114 extracts of P.falciparum infected red blood cells separated on 10% SDS-PAGE, electrophoretically transferred to nitrocellulose and probed with either (A) pooled adult PNG sera, or (B) affinity purified poly clonal human antibodies. Lanes are, (A), total unfractionated material, (B), aqueous phase, (C), Triton X-114 phase, (D), insoluble material.

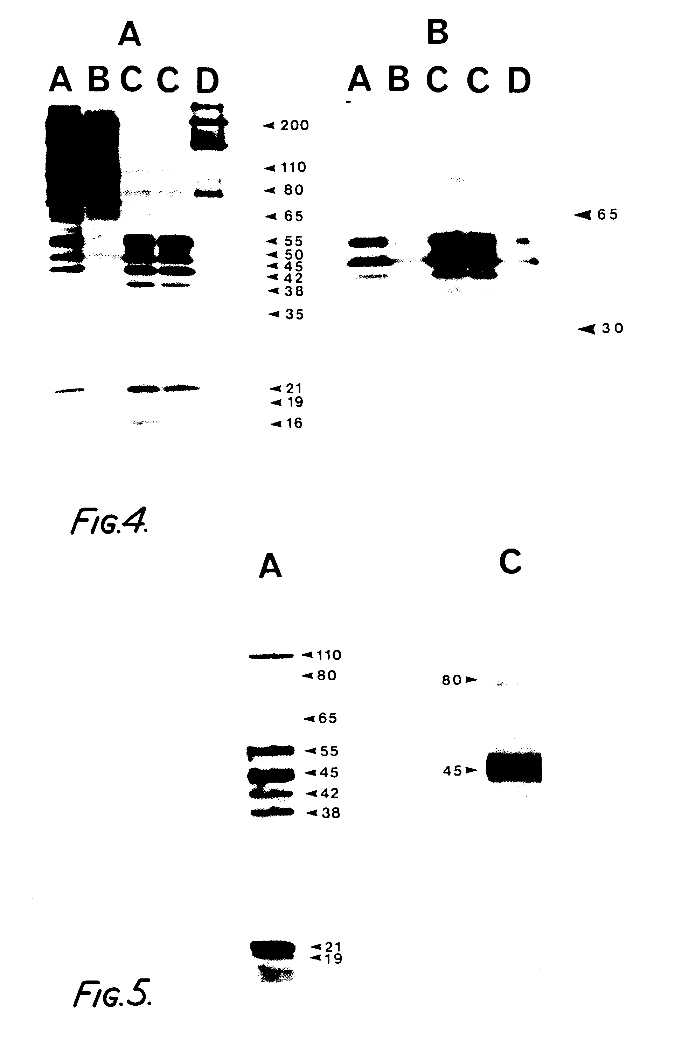

- Figure 5:: Human antibodies from PNG adult sera affinity purified on lawns of λgt11-Amp3 cDNA clones which expressed immunoreactive polypeptides of native Triton X-114 extractable antigens in the molecular weight range 30-36 kD. (A) Control strip of Triton X-114 phase antigens probed with pooled adult PNG sera and iodinated Protein A. (C) Human antibodies affinity purified on Ag513 probed on a duplicate filter strip of the control.
- Figure 6:: Sequence of Ag513 with a predicted translation of the single long open reading frame.

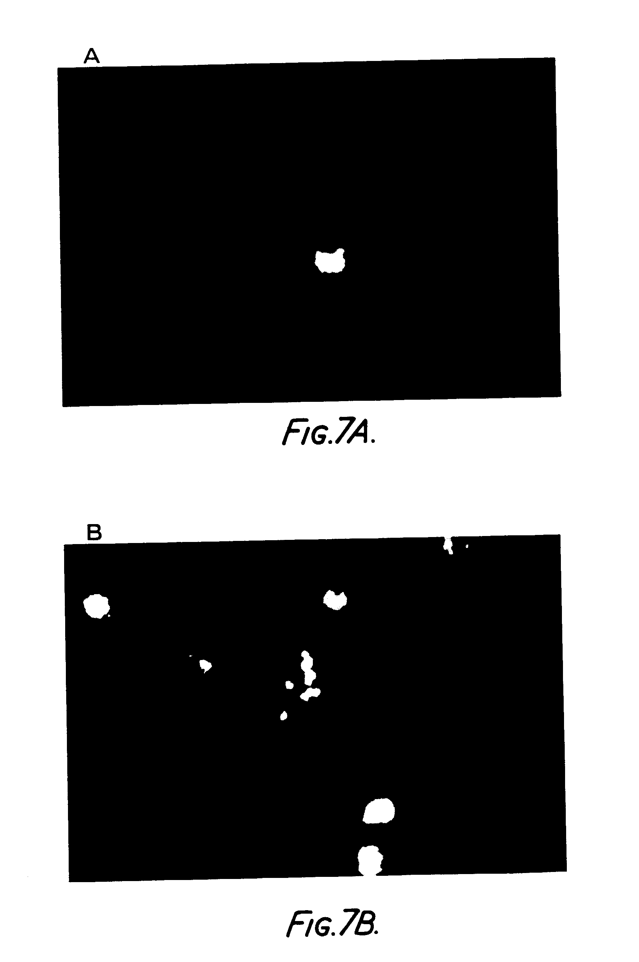

- Figure 7:: Immunofluorescence location affinity purified human antibodies to Ag513 used in indirect immunofluorescence assays on asynchronous blood films: schizont (A) and matured merozoite (B).

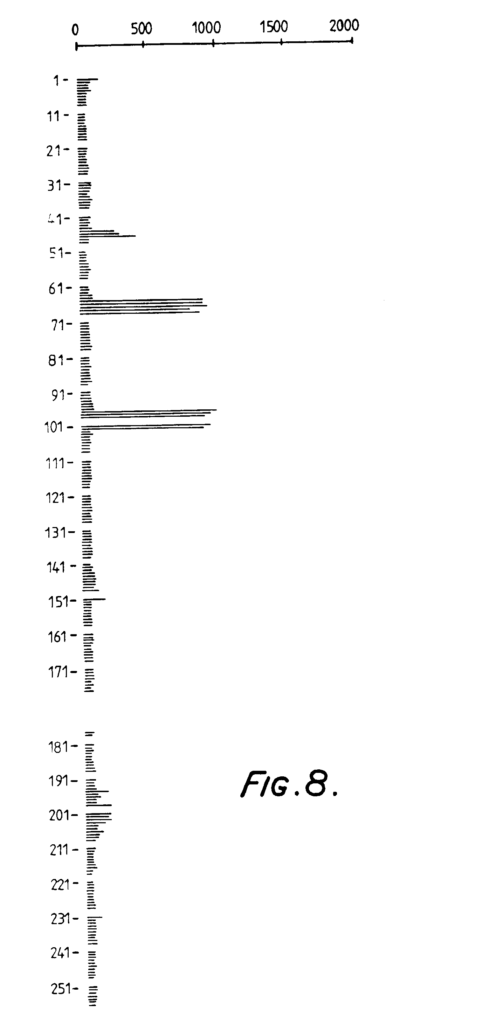

- Figure 8:: Scan of monoclonal antibody PG10/48 on the predicted amino acid sequence of the insert of Ag513.

### A subset of P.falciparum antigens partition into Triton X-114.

When sera from individuals repeatedly infected with P.falciparum are used to probe immunoblots of P.falciparum antigens fractionated by phase separation in Triton X-114 several putative integral membrane protein on antigens are identified. The most dominant of these had relative molecular masses (Mr) of 21,000, 35,000, 42,000, 50,000 and 55,000 as determined SDS-PAGE analysis. When the same antigen extracts were probed with antisera raised against or affinity purified on a number of cloned P.falciparum antigens it was shown that the Mr 21,000 Triton X-114 soluble antigen was the circumsporozoite protein related antigen (CRA). This molecule is known to have a 26 amino acid hydrophobic sequence typical of integral membrane proteins (47), confirming that the procedures selects for such molecules. None of the other Triton X-114 soluble antigens appeared to correspond to any of the many P.falciparum antigens that have been previously characterised.

In order to obtain purified antibodies specific for the set of proteins of Mr 35-55,000 Triton X-114 extracts of P.falciparum were electrophoretically fractionated and transferred to nitrocellulose. The appropriate region of the nitrocellulose filter was then excised and used as an adsorbent for affinity purification of the corresponding antibodies from human serum (Materials and Method). The purified antibodies were tested for specificity by reacting them with fractionated P.falciparum proteins. It is clearly evident in Fig.4B that these purified antibodies react almost exclusively with those Mr 35-55,000 antigens that selectively partition into the Triton X-114 phase.

In order to isolate clones expressing these antigens, a library of λAmp3 clones expressing P.falciparum cDNA sequences was screened with the affinity-purified antibodies. Ten positive lysogenic clones that were detected by this procedure were replated for single colonies.

### Selected colonies correspond to Triton X-114 soluble antigens.

Antibodies affinity purified on lawns of selected clones were used in colony immunoassays on the array of ten clones and to probe immunoblots of parasite antigens. Three of the clones were found to be siblings encoding the Mr 45,000 antigen from the Triton X-114 phase. The most immunoreactive was chosen as the type clone and designated Ag513.

Analysis of clones by SDS-PAGE followed by protein staining or immunoblotting showed that Ag513 produced a single polypeptide of Mr 42,000, very close to the size of the native parasite antigen (Fig.5). Immuno sera were absorbed with sonicates of the most immunoreactive cDNA clones of Ag513 and then used to probe immunoblots of Triton X-114 extracts of P.falciparum. Sonicates of the cDNA clones totally removed all detectable antibody reactivity to the corresponding parasite antigens. Thus the cDNA clones apparently encode the dominant naturally immunogenic epitopes in these antigens.

### Stage and strain specificity.

Stage-specific parasite preparations were Triton X-114 extracted, fractionated by SDS-PAGE and immunoblots probed with PNG sera. The antigen corresponding to Ag513 was present in all stages of the parasite life cycle, the abundance of the antigen ranging from at least abundant in the asexual ring stage to most abundant in late schizont preparations. In all stages it is totally Triton X-114 soluble. Triton X-114 extracts were prepared from five strains of P.falciparum giving asychronously in culture. The antigen was present in all strains and there were no apparent strain-related differences in the size or immunoreactivity of the antigen.

### Nucleotide and amino acid sequence of Ag513.

The nucleotide sequence of Ag513 together with the predicted amino acid sequence is Fig.6. It contains only one long open reading frame, commencing at base 94 and continuing to base 886. The entire fragment was 1145 bases in length. The AT content of the 5' sequence preceding base 94 is greater than 92%, whereas the AT content of the predicted coding region is approximately 65%. Increased AT content in non-coding regions has been previously documented for several other P.falciparum antigens, suggesting that the methionine indicated is the actual initiation codon. The 5' end of the coding sequence contains a stretch of 15 uncharged and hydrophobic amino acids corresponding at residue 6, is most likely the core of a signal which sequence. The sequence encodes two identical copies of a 32 amino acid unit arranged in tandem, beginning at base 271 and continuing to base 462. The stop codon at position 886 is preceded by a stretch of 17 hydrophobic amino acids before the sequence again resumes at AT content in excess of 80%. This hydrophobic sequence is presumed to be an integral membrane protein anchor sequence although there is no charge residue at its C-terminal end. The translated protein would have a total molecular weight of 27,860 daltons. The protein is rich in the hydroxy amino acids serine and threonine which comprises approximately 28% of the sequence.

### Ag513 corresponds to a putative merozoite surface antigen.

Affinity purified human antibodies to Ag513 were prepared as described, concentrated, and used in indirect immunofluorescence assays on asynchronous blood films. The fluorescence pattern observed was consistent with localisation of the antigen on the parasite membrane in trophozoites. In later stages of parasite development, i.e. the schizont and mature merozoite, the fluorescent pattern was consistent with localisation of Ag513 to the merozoite surface (Fig.7).

### The antigen encoded by Ag513 correspond to QF122.

The size and physical characteristics of the antigen encoded by Ag513 suggested that it may correspond to QF122. This was confirmed by colony immunoassays in which monoclonal antibodies to QF122 reacted strongly with Ag513 but not other P.falciparum antigen-expressing cDNA clones.

Further confirmation of this relationship was obtained by detailed epitope mapping using the procedure of Geysen et.al. (37). An overlapping set of 8 amino acid peptides covering the complete coding sequence in the Ag513 cDNA insert were probed with three monoclonal antibodies to QF122. Epitopes were identified in the Ag513 sequence for each of the monoclonal antibodies. The scan with Mab 8G10/48 is shown in Fig.8. The dominant epitope recognised by this monoclonal antibody contains the sequence STNS and occurs twice as it is within the 32 amino acid repeat found in Ag513. The monoclonal antibody reacts with a second related sequence (SNTNSV) on the N-terminal side of the 32 residue repeats.

### DISCUSSION

This Example shows the temperature dependent Triton X-114 detergent separation of integral membrane protein antigens of P.falciparum. One of the antigens that partitioned to the Triton X-114 phase was identified as CRA, a well characterised antigen of known primary structure which is typical of an integral membrane protein. The other dominant antigens that partition into the Triton X-114 clustered into the Mr 40-55,000 range and apparently did not correspond to antigens that had been previously cloned. These proteins, after transfer to nitrocellulose were used to affinity purify polyclonal monospecific human antibodies which were used to isolate a clone designated Ag513, corresponding to a membrane protein, from a λAmp3 cDNA expression library. Antibodies to Ag513 reacted with a Triton X-114 soluble protein of Mr 45,000 which was present in all stains of P.falciparum examined and present in all of the asexual life cycle stages. Indirect immunofluorescent microscopy with these antibodies gave strong staining on mature stages with a grape-like pattern on segmented schizonts, a pattern characteristic of antibodies to merozoite surface antigens. The primary structure of the Ag513 related antigen has been deduced from the nucleotide sequence of the full length cDNA sequence. Consistent with the anticipated structure of an integral membrane protein the deduced amino acid sequence included, at the N-terminus, a typical signal sequence with a hydrophobic core of 15 residues and, at the C-terminus a second very hydrophobic sequence, 21 residues in length and presumed to be a membrane anchor.

### EXAMPLE 3

This Example describes a further sequence coded for by P.falciparum which is recognised by monoclonal antibodies 8G10/48 and 9E3/48.

### Materials and Methods

### Screening of λgt11 recombinant DNA libraries and nucleic acid sequence determination

A clone was obtained from a cDNA expression library constructed in λgt11 Amp3 (32) using the FCQ-27/PNG strain of Plasmodium falciparum, (33). Monoclonal antibodies selected for screening the cDNA library were those which produced strong signals in an immunoradiometric assay (30). This selection bias increased the likelihood of identifying positive recombinants in the colony assay described below.

Recombinant cultures were plated onto 82cm nitrocellulose filters at a plating density of 3-4 x 10³ cells per filter and grown on Luria-Bertani agar plates for 8h at 32°C. Colonies were replicated and grown for a further 2h at 32°C. The colonies were heat-induced at 42°C for 1h and lysed by placing the filters in 50mM Tris/HCl (pH 7.5) containing 150mM NaCl, 5mM MgCl₂, 0.25% (w/v) gelatin, 40 µg ml⁻¹ lysozyme and 1 µg ml⁻¹ deoxyribonuclease 1. After 30min the filters were washed for 2h at 20°C in PBS containing 5% (w/v) low fat milk powder and then rinsed with PBS.

Filters were incubated overnight at 4°C with 10ml of hybridoma supernatants and washed three times for 10min at 20°C with PBS containing 5% (w/v) low-fat milk powder. Following a brief wash with PBS, the filters were incubated for 2h at 20°C in PBS containing [¹²⁵I] goat anti-mouse Ig (40 µCi µg⁻¹, 0.5 µCi ml⁻¹) and 1% (w/v) bovine serum albumin. They were washed three times in PBS containing 5% low-fat milk powder, dried and autoradiographed for 2 to 3 days.

Standard techniques (34) were used to purify DNA from antibody-positive clones, digest the nucleic acids with EcoRI restriction endonuclease and recover the cDNA insert following agarose gel electrophoresis of the digest fragments. EcoRI fragments were cloned into M13 mp18 and mp19 (35) and the nucleic acid sequence determined by the dideoxy chain-termination procedure (36).

### Octapeptide synthesis and epitope determination.

Scanning for antibody-reactive peptides was performed as described by Geysen et.al. (37). Synthetic peptides were coupled via a peptide-like spacer to polyethylene rods to which a linear polymer of acrylic acid had been attached by radiation grafting. Overlapping octapeptides were synthesised consecutively using solid phase peptide synthesis based upon nucleic acid sequence data. Following removal of the t-butyloxycarbonyl group from the final amino acid, peptides were acylated and de-protected to provide material of sufficient purity to enable ELISA testing for the specific binding of monoclonal antibody to each octapeptide (38). A complete "replacement" set of 120 peptides was made based upon the sequence STNSGI where each amino acid was substituted in turn by the other naturally occurring amino acids.

### LEGENDS TO FIGURES

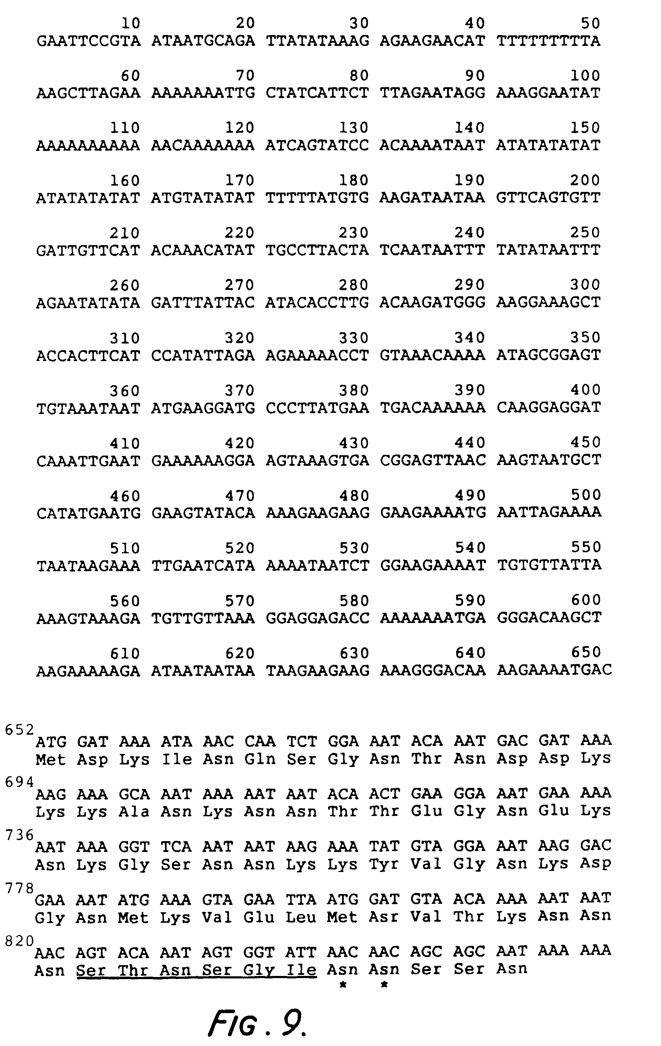

- Figure 9:: Nucleotide sequence of the cDNA clone recognised by monoclonal antibodies 8G10/48 and 9E3/48. The deduced amino acid sequence and putative epitope (underlined) are shown in addition to potential sites for N-glycosylation, denoted by *.

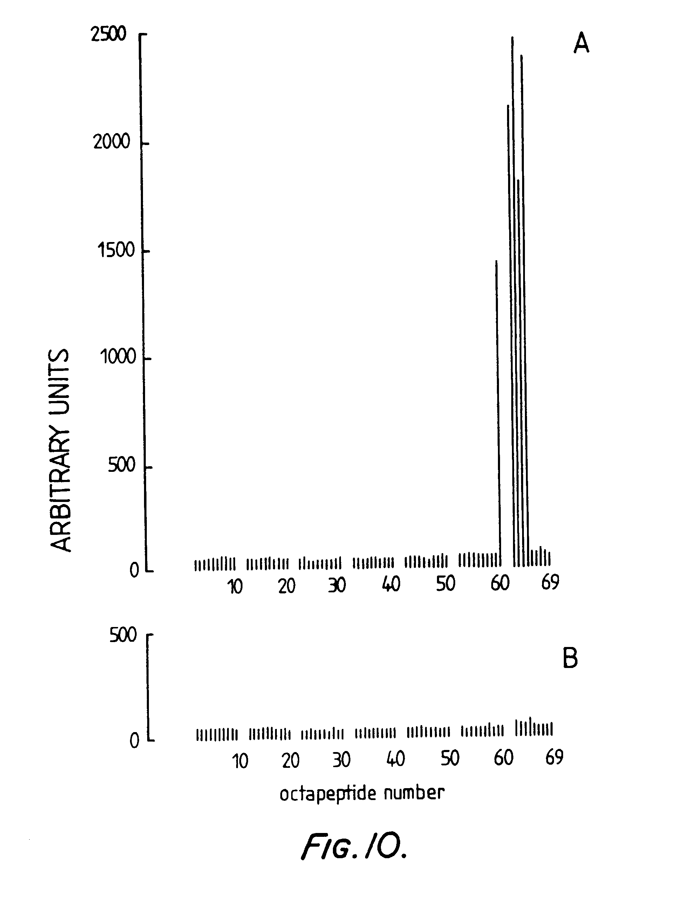

- Figure 10:: Rod-coupled octapeptides were reacted with (A) 1/10,000 dil. of 8G10/48 hybridoma supernatant, (B) 1/100 dil. of control monoclonal antibody supernatant. Bound antibody was detected by ELISA using horseradish peroxidase-conjugated anti-mouse Ig. X-axis: arbitrary units: Y-axis: figures represent the amino acid position of the primary octapeptide amino acid based upon the sequence shown in Fig.9.

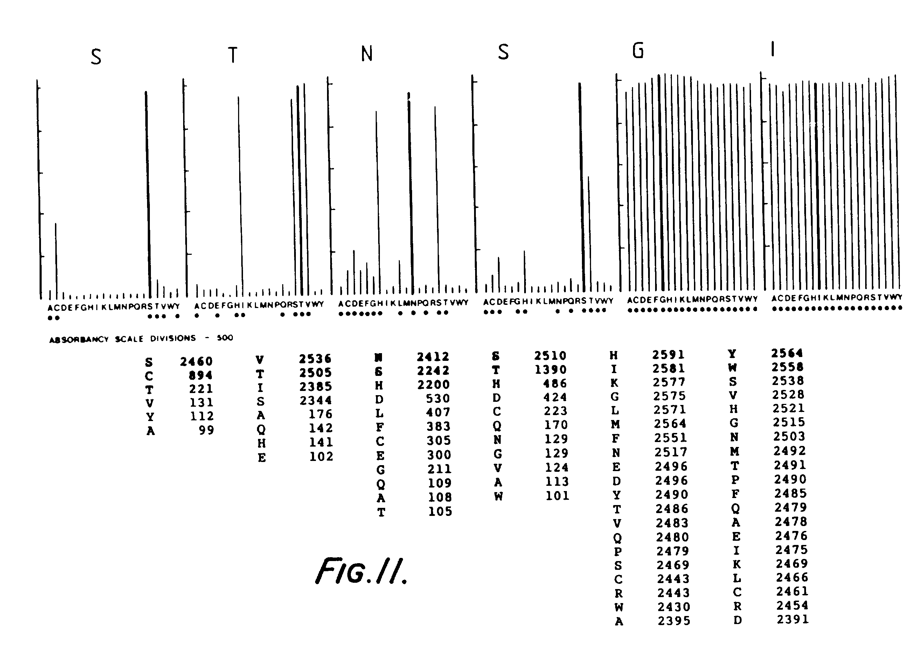

- Figure 11:: Replacement net analysis of the sequence STNSGI using 8G10/48 at a 1/1000 dilution.

### Results and Discussion

### Identification and characterisation of clone CL2122

8G10/48 and 9E3/48 were used to screen an expression library constructed in λgt11 Amp3 to select for recombinants expressing cDNA fragments of Plasmodium falciparum. One clone (designated CL2122) was recognised by both antibodies. The EcoRI restriction fragment is 855 nucleotides in length followed by a region of poly (A) nucleotides (Fig.9). Nucleotide repeats which are characteristic of other Plasmodium falciparum genes are absent from this clone. The sequence apparently is not expressed as a fusion polypeptide coupled to the β-galactosidase gene product (data not shown). Initiation signals characteristic of internal sites for RNA synthesis (38a) are present from bases 592 to 627 and a plausible polypeptide encoded by this sequence is presented (Fig.9). A change in codon usage from that of typical non-coding DNA sequences to coding sequences characteristic of translated regions of malarial genes (40) in the vicinity of base 650 supports this interpretation.

This sequence is rich in Asn and Lys residues (45% of total amino acids), contains several potential sites for N-glycosylation and does not code for part of QF122. Many Plasmodium falciparum proteins contain lysine-rich sequences for which the predominant codon usage is AAA. The possibility thus arises that the reverse transcriptase used during construction of the cDNA library may have been primed by oligo d(T) on an internal lysine-rich sequence commencing at base 856 with the implication that only the amino terminus of the native protein has been cloned.

### Identification of the epitope recognised by 8G10/48 and 9E3/48.

Overlapping octapeptides corresponding to the derived amino acid sequence shown in Fig.9 were synthesised and examined for reactivity with hybridoma supernatants of monoclonal antibodies 8G10/48 and 9E3/48. Five octapeptides spanning the sequence Lys Asn Asn Asn Ser Thr Asn Ser Gly Ile Asn Asn produced strong positive ELISA signals using both antibodies at supernatant dilutions to 1/10,000 (Fig.10). Negative results were obtained using 9 other monoclonal antibodies at 1/100 dilution of hybridoma supernatant (Fig.10). The epitope recognised by the monoclonal antibodies is the sequence common to these five octapeptides, namely STNS.

### Replacement net analysis of the epitope.

The binding of antibodies 8G10/48 and 9E3/48 to every possible single amino acid substitution of the sequence STNSG1 is shown in Figure 11 for 8G10/48 when assayed at a 1/1000 dilution. 9E3/48 gave similar results. These data confirm the sequence STNS as the epitope recognised by these monoclonal antibodies and show that some related peptides will bind these antibodies, especially peptides where:
1. The first S is substituted by C, T or V;
2. T is substituted by I, S or V;
3. N is substituted by C,D,E,F,G,H,L or S;
4. The second S is substituted by C,D,H or T.

### DISCUSSION OF EXAMPLES 1 to 3

The antigens described in Examples 1 and 2 above are the same antigen, having the same Western blotting patterns, immunoprecipitation and labelling characteristics and identical immunofluorescence patterns. Furthermore, it has been shown that monoclonal antibodies which define QF122 described in Example 1 bind to sequences which are coded for by the insert in Ag513 described in Example 2. The immunofluorescence patterns are consistent with a merozoite surface location and the immunoelectron microscopy has localised an antigen on the surface of merozoites. While it is possible that the localisation by electronmicroscopy could be due to the reaction of the monoclonal antibody with some other cross-reacting antigen, the consistency of the immunofluorescence patterns obtained with all three monoclonal antibodies and the human serum suggests that this localisation is due tobinding to the antigen QF122.

The surface location of antigen QF122 would give a plausible explanation for the inhibition of parasite growth by monoclonal antibodies 8G10/48 and 9E3/48. However, the finding that there are other malarial sequences capable of coding for proteins containing the epitope recognised by monoclonal antibodies 8G10/48 and 9E3/48 suggests that other mechanisms of growth inhibition might be important. Furthermore, since the mice that were used for the production of these monoclonal antibodies were immunised with whole parasitized red cells, it cannot be determined whether it was QF122 or some other protein containing the sequence STNS (for example, the putative sequence coded for by CL2122 in Example 3), or even some related sequence, which elicited this protective response.

### REFERENCES

- 1.: Mitchell, G.H., Butcher, G.A., Langhorn, J. and Cohen, S. (1977) Clin.Exp.Immunol. 28, 276-279.
- 2.: Mitchell, G.H., Richards, W.H., Butcher, G.A. and Cohen, S. (1977) Lancet 1, 1335-1338.
- 3.: Siddiqui, W.A. (1977), Science 197, 388-389.
- 4.: Freeman, R.R. and Holder, A.A. (1983) J.Exp.Med. 158, 1647-1653.
- 5.: Heidrich, H.G., Strych, W., and Mrema, J.E.K. (1983). Z.Parasitenkd 69, 715-725.
- 6.: Reese, R.T., Motyl, M.R. and Hofer-Warbinek, R. (1981). Am.J.Trop.Med.Hyg. 30, 1168-1178
- 7.: Perrin, L.H., Dayal, R., and Reider, H. (1981) Trans.R.Soc.Trop.Med.Hyg. 75, 163-165.
- 8.: Howard, R.F. and Reese, R.T. (1984) Molec.Biochem.Parasitol. 10, 319-334.
- 9.: Holder, A.A., Lockyer, M.J., Odink, K.G., Sandhu, J.S., Rivercos-Moreno, V., Nicholls, S.C., Hillman, Y., Davey, L.S., Tizard, M.L.V., Schwarz, R.T. and Freeman, R.R. (1985), Nature, 317, 270-273.
- 10.: Holder, A.A. and Freeman, R.R. (1984), J.EXD.Med. 160, 624-629.
- 11.: Lyon, J.A., Geller, R.H., Haynes, J.D., Chulay, J.D. and Weber, J.L. (1986) Proc.Natl.Acad.Sci. (USA). 83, 2989-2993.
- 12.: Perrin, L.H., Merkli, B., Loche, M., Chizzolini, C., Smart, J. and Richle, R. (1984) J.Exp.Med. 160, 441-451
- 13.: Mackay, M. Goman, M., Bone, N., Hyde, J.E., Scaife, J., Certa, U., Stunnenberg, H. and Bujard, H. (1985) Embo.J. 4, 3823-3829.
- 14.: McBride, J.S., Newbold, C.I., and Anand, R. (1985) J.Exp.Med. 161, 160-180.
- 15.: Weber, J.L., Leininger, W.M. and Lyon, J.A. (1986) Nucl.Acids Res. 14, 3311-3323.
- 16.: Holder, A.A. and Freeman, R.R. (1981) Nature 294, 361-364.
- 17.: Epstein, N., Miller, L.H., Kaushel, D.C., Udeinya, I.J., Rener, J., Howard, R., Asofsky, R., Aikawa, M. and Hess, R.L., (1981), J.Immunol. 127, 212-217.
- 18.: Kilejian, A. (1980), Proc.Natl.Acad.Sci. (USA) 77, 3695-3699.
- 19.: Schofield, D.L. (1985). Plasmodium falciparum: Inhibitory antibodies and antigenic diversity. Ph.D. Thesis. Dept.Biochemistry, University of Queensland.
- 20.: Epping, R.J., Dharmkrong-At, A., Jelacic, S. and Upcroft, J. (1986), Internat. Congress of Parasitol. VI, 205.
- 21.: Chen, P., Lamont, G., Elliot, T., Kidson, C., Brown, G., Stace, J. and Alpers, M. (1980) Southeast Asian J.Trop.Mod.Pub. Health 11, 435-440.
- 22.: Trager, W., and Jensen, J.B. (1976) Science 193, 673-675.
- 23.: Myler, P., Saul, A., Mangan, T., and Kidson, C. (1982) Aust.J.Exp.Med.Sci. 1, 83-89.
- 24.: Galfre, G., Hove, S.C., Milstein, C., Butcher, G.W. and Howard, J.C. (1977) Nature (Lond.) 266, 500-552.
- 25.: Schofield, L., Saul, A., Myler, P. and Kidson, C. (1982) Infect.Immun. 28, 892-897.
- 26.: Fraker, P.J. and Speck, J.C.Jr. (1978) Biochem.Biophys.Res.Comm. 80, 849-857.
- 27.: Lyon, J.A. and Haynes, J.D. (1986) J.Immunol. 136, 2245-2251.
- 28.: Laemmli, U.K. (1970) Nature 227, 680-685.
- 29.: Towbin, H., Staehelin, T. and Gordon, J. (1979). Proc.Natl.Acad.Sci.(USA) 76, 4350-4352.
- 30.: Schofield, L., Bushell, G.R., Cooper, J.A., Saul, A.J., Upcroft, J.A. and Kidson, C. (1986) Molec.Biochem.Parasitol. 18, 183-185.
- 31.: Ramasamy, R. and Reese, R.T. (1985) J.Immunol. 134, 1952-1955.
- 31a.: Haldar, K., et.al. (1985). J.Biol.Chem. 260, 4969.
- 32.: Young, N. and Davis, R.W. (1983), Proc.Natl. Acad.Sci. (USA), 80, 1194-1198.
- 33.: Kemp, D.J., Coppel, R.L., Cowman, A.F., Saint, R.B., Brown, G.V. and Anders, R.F. (1983) Proc.Natl.Acad.Sci. (USA) 80, 3787-3791.
- 34.: Maniatis, T., Fritch, E.F. and Sambrook, J. (1982) Molecular Cloning (Cold Spring Harbor Laboratory, Cold Spring Harbor, N.Y.).
- 35.: Yanisch-Perron, C., Vieira, J. and Messing, J. (1985) Gene 33, 103-119.
- 36.: Sanger, F., Nicklen, S. and Coulsen, A.R. (1977) Proc.Natl.Acad.Sci (USA). 74, 5463-5467.
- 37.: Geysen,H.M., Meloen, R.H. and Barteling, S.J. (1984). Proc.Natl.Acad.Sci.(USA). 81, 3998-4002.
- 38.: Rodda, S.J., Geysen, H.M., Mason, T.J. and Schoofs, P.G. (1986) Molec. Immunol. 23, 603-610.
- 38a.: Efstratiadis, A., Posakony, J.W., Maniatis, T., Lawn, R.M., O'Connel, C., Spritz, R.A., DeRiel, J.C., Forget, B.G., Weissman, S.M., Slighton, J.L., Blechl, A.E., Smithies, O., Baralle, F.E., Shoulders, C.C., and Proudfoot, N.J. (1980), Cell 21, 653-668.
- 39.: Corcoran, L.M., Forsyth, K.P., Bianco, A.E., Brown, G.V. and Kemp, D.J. (1980) Cell 44, 87-95.
- 40.: Saul. A. and Battistutta, D., (1987) Molec.Biochem.Parasitol. (In press).
- 41.: Saul, A., Myler, P., Schofield, L. and Kidson, C. (1984). Parasite Immunology. 6, 39-50.
- 42.: Coppel, R.L.,Saint, R.B., Stahl, H.D., Langford, C.J., Brown, G.V., Anders, R.F. and Kemp, D.J. (1985). Exp.Parasitol. 60, 82-89.
- 43.: Bordier, C. (1981). J.Biol.Chem. 256, 1604-1607.
- 44.: Beal, J.A., Mitchell, G.F. (1986), J.Immunol. Methods. 86, 217-223.
- 45.: Anders, R.F., Coppel, R.L., Brown, G.V., Saint, R.B., Cowman, A.F., Lingelbach, K.R., Mitchell, G.F. and Kemp, D.J. (1984). Molec. Biol.Med. 2, 177-192.
- 46.: Messing, J. and Vieira, J. (1982). Gene 19, 269-276.
- 47.: Hope, I.A., Mackay, M., Hyde, J.E. (1985). Nucl.Acids Res. 13, 269-279.

## Claims (Claims for the following Contracting State(s): BE, CH, LI, DE, FR, GB, IT, LU, NL, SE)

1. An isolated merozoite surface antigen of the asexual blood stages of Plasmodium falciparum, other than the FVO and Geneva isolates of P.falciparum, which is characterised by:
(i) having an apparent molecular weight in the range of approximately 41 kDa to 53 kDa by reducing SDS PAGE;
(ii) being a glycoprotein incorporating myristic acid;
(iii) being present firstly as a diffuse cytoplasmic localisation and in mature schizonts on the surface membrane of merozoites;
(iv) being recognised by monoclonal antibodies against the asexual blood stages of P.falciparum which inhibit parasite growth in vitro; and
(v) when isolated from strain FCQ-27/PNG, having the amino acid sequence set out in Figure 6;
and which does not derive from the 195 KDa precursor to the major merozoite surface antigen (PMMSA);
or an antigenic fragment of said antigen that elicits a specific immune response in a host.

2. An isolated antigen or antigenic fragment according to claim 1, wherein the antigen has the amino acid sequence set out in Figure 6.

3. An isolated antigen or antigenic fragment according to claim 1 or claim 2 which is characterised by inclusion in the amino acid sequence thereof of the <sequence Ser-Thr-Asn-Ser (STNS) or the sequence Ser-Asn-Thr-Asn-Ser-Val (SNTNSV)>.

4. An isolated antigen or antigenic fragment of Plasmodium falciparum according to any one of claims 1 to 3 which is recognised by the monoclonal antibody 8G10/48 produced by the cell line deposited with the European collection of Animal Cell Cultures, Porton Down, under No. 87081010.

5. Use of an antigen or antigenic fragment according to any one of claims 1 to 4, for the preparation of a vaccine composition for actively immunising a host against Plasmodium falciparum.

6. A vaccine composition comprising an antigen or antigenic fragment according to any one of claims 1 to 4, and a pharmaceutically acceptable carrier or diluent.

7. A composition according to claim 6, further comprising an adjuvant.

8. A composition according to claim 6, wherein the antigen has the sequence set out in Figure 6 or an antigenic fragment thereof that elicits a specific immune response in a host.

9. A recombinant DNA molecule comprising a nucleotide sequence which is capable of being expressed as polypeptide capable of inducing an immune response to an antigen or antigenic fragment according to any one of claims 1 to 4, or a recombinant cloning vehicle or vector or a host cell comprising a said recombinant DNA molecule.

10. A synthetic polypeptide prepared by expression of all or a portion of a nucleotide sequence according to claim 9.

11. A vaccine composition comprising a synthetic polypeptide according to claim 10.

## Claims (Claims for the following Contracting State(s): AT)

1. A process for the preparation of an isolated merozoite surface antigen of the asexual blood stages of Plasmodium falciparum, other than the FVO and Geneva isolates of P.falciparum, said antigen being
characterised by:
(i) having an apparent molecular weight in the range of approximately 41 kDa to 53 kDa by reducing SDS PAGE;
(ii) being a glycoprotein incorporating myristic acid;
(iii) being present firstly as a diffuse cytoplasmic localisation and in mature schizonts on the surface membrane of merozoites;
(iv) being recognised by monoclonal antibodies against the asexual blood stages of P.falciparum which inhibit parasite growth in vitro; and
(v) when isolated from strain FCQ-27/PNG, having the amino acid sequence set out in Figure 6;
and which does not derive from the 195 KDa precursor to the major merozoite surface antigen (PMMSA) ;
or an antigenic fragment of said antigen that elicits a specific immune response in a host, wherein said process comprises the isolation of said antigen or a fragment thereof from P.falciparum cells or from recombinant cells capable of expressing the antigen.

2. A process according to claim 1, wherein said antigen has the amino acid sequence set out in Figure 6.

3. A process according to claim 1 or claim 2, said antigen being characterised by inclusion in the amino acid sequence thereof of the (sequence Ser-Thr-Asn-Ser (STNS) or the sequence Ser-Asn-Thr-Asn-Ser-Val (SNTNSV)).

4. A process according to any one of claims 1 to 3 in which said antigen or antigenic fragment is recognised by the monoclonal antibody 8G10/48 produced by the cell line deposited with the European collection of Animal Cell Cultures, Porton Down, under No. 87081010.

5. Use of an antigen or antigenic fragment prepared according to any one of claims 1 to 4, for the preparation of a vaccine composition for actively immunising a host against Plasmodium falciparum.

6. A process for the preparation of a vaccine composition comprising admixing an antigen or antigenic fragment according to any one of claims 1 to 4, with a pharmaceutically acceptable carrier or diluent.

7. A process for the preparation of a vaccine composition according to claim 6, further comprising adding an adjuvant.

8. A process for the preparation of a vaccine composition according to claim 6 or claim 7, wherein said antigen has the sequence set out in Figure 6 or an antigenic fragment thereof that elicits a specific immune response in a host.

9. A method for the expression of a recombinant DNA molecule, characterised in that said molecule comprises a nucleotide sequence which is capable of being expressed as a polypeptide capable of inducing an immune response to an antigen or antigenic fragment according to any one of claims 1 to 4.

10. A method for preparing a recombinant cloning vehicle or vector or host cell comprising introducing a recombinant DNA molecule as defined in claim 9 into a cloning vehicle, vector or host cell.

11. A process for the preparation of a synthetic polypeptide comprising the expression of all or a portion of a nucleotide sequence as defined in claim 9.

12. A process for the preparation of a vaccine composition comprising admixing a synthetic polypeptide according to claim 11 with a pharmaceutically acceptable diluent.

## Patentansprüche (Patentansprüche für folgende(n) Vertragsstaat(en): BE, CH, LI, DE, FR, GB, IT, LU, NL, SE)

1. Isoliertes Merozoiten-Oberflächenantigen der asexuellen Blutstufen von Plasmodium falciparum mit Ausnahme der FVO- und Geneva-Isolate von P.falciparum, welches dadurch gekennzeichnet ist, daß es
(i) ein scheinbares Molekulargewicht im Bereich von etwa 41 kDa bis 53 kDa im Rahmen einer reduzierenden SDS-PAGE besitzt;
(ii) ein Myristinsäure umfassendes Glycoprotein ist;
(iii) zunächst als diffuse Cytoplasmalokalisation und in reifen Schizonten auf der Oberflächenmembran von Merozoiten vorhanden ist;
(iv) durch monoklonale Antikörper gegen die asexuellen Blutstufen von P.falciparum, die ein Parasitenwachstum in vitro hemmen, erkannt wird, und
(v) bei Isolierung aus dem Stamm FCQ-27/PNG die in Fig. 6 dargestellte Aminosäuresequenz aufweist,
und nicht von dem 195 kDa-Vorläufer des Hauptmerozoitenoberflächenantigens (PMMSA) herrührt,
oder ein antigenes Fragment des betreffenden Antigens, das in einem Wirt eine spezifische Immunantwort hervorruft.

2. Isoliertes Antigen oder antigenes Fragment nach Anspruch 1, wobei das Antigen die in Fig. 6 dargestellte Aminosäuresequenz aufweist.

3. Isoliertes Antigen oder antigenes Fragment nach Anspruch 1 oder 2, welches durch Einbau der Sequenz Ser-Thr-Asn-Ser (STNS) oder der Sequenz Ser-Asn-Thr-Asn-Ser-Val (SNTNSV) in seine Aminosäuresequenz gekennzeichnet ist.

4. Isoliertes Antigen oder antigenes Fragment von Plasmodium falciparum nach einem der Ansprüche 1 bis 3, welches durch den monoklonalen Antikörper 8G10/48, welcher durch die bei der Europäischen Sammlung von Tierzellkulturen, Porton Down, unter der Nr. 87081010 hinterlegte Zellinie gebildet wird, erkannt wird.

5. Verwendung eines Antigens oder antigenen Fragments nach einem der Ansprüche 1 bis 4 zur Zubereitung eines Impfstoffs zur aktiven Immunisierung eines Wirts gegen Plasmodium falciparum.

6. Impfstoff, umfassend ein Antigen oder ein antigenes Fragment nach einem der Ansprüche 1 bis 4 sowie ein(en) pharmazeutisch akzeptablen (akzeptables) Träger oder Verdünnungsmittel.

7. Impfstoff nach Anspruch 6, weiterhin enthaltend ein Adjuvans.

8. Impfstoff nach Anspruch 6, wobei das Antigen die in Fig. 6 dargestellte Sequenz aufweist, oder ein antigenes Fragment desselben, welches in einem Wirt eine spezifische Immunantwort hervorruft, ist.

9. Rekombinantes DNA-Molekül, umfassend eine Nucleotidsequenz mit der Fähigkeit, als Polypeptid, welches eine Immunantwort auf ein Antigen oder ein antigenes Fragment nach einem der Ansprüche 1 bis 4 hervorzurufen vermag, exprimiert zu werden, oder ein rekombinantes Klonierungsvehikel oder ein Vektor oder eine Wirtzelle, der (die) das rekombinante DNA-Molekül enthält.

10. Synthetisches Polypeptid, hergestellt durch Expression der gesamten oder eines Teils einer Nucleotidsequenz nach Anspruch 9.

11. Impfstoff, umfassend ein synthetisches Polypeptid nach Anspruch 10.

## Patentansprüche (Patentansprüche für folgende(n) Vertragsstaat(en): AT)

1. Verfahren zur Herstellung eines isolierten MerozoitenOberflächenantigens der asexuellen Blutstufen von Plasmodium falciparum mit Ausnahme der FVO- und Geneva-Isolate von P.falciparum, welches dadurch gekennzeichnet ist, daß es
(i) ein scheinbares Molekulargewicht im Bereich von etwa 41 kDa bis 53 kDa im Rahmen einer reduzierenden SDS-PAGE besitzt;
(ii) ein Myristinsäure umfassendes Glycoprotein ist;
(iii) zunächst als diffuse Cytoplasmalokalisation und in reifen Schizonten auf der Oberflächenmembran von Merozoiten vorhanden ist;
(iv) durch monoklonale Antikörper gegen die asexuellen Blutstufen von P.falciparum, die ein Parasitenwachstum in vitro hemmen, erkannt wird, und
(v) bei Isolierung aus dem Stamm FCQ-27/PNG die in Fig. 6 dargestellte Aminosäuresequenz aufweist,
und nicht von dem 195 kDa-Vorläufer des Hauptmerozoitenoberflächenantigens (PMMSA) herrührt,
oder eines antigenen Fragments des betreffenden Antigens, das in einem Wirt eine spezifische Immunantwort hervorruft, wobei das Verfahren ein Isolieren des Antigens oder eines Fragments hiervon aus P.falciparum-Zellen oder aus rekombinanten Zellen mit der Fähigkeit zur Expression des Antigens umfaßt.

2. Verfahren nach Anspruch 1, wobei das Antigen die in Fig. 6 dargestellte Aminosäuresequenz aufweist.

3. Verfahren nach Anspruch 1 oder 2, wobei das Antigen durch den Einbau der Sequenz Ser-Thr-Asn-Ser (STNS) oder der Sequenz Ser-Asn-Thr-Asn-Ser-Val (SNTNSV) in seine Aminosäuresequenz gekennzeichnet ist.

4. Verfahren nach einem der Ansprüche 1 bis 3, wobei das Antigen oder antigene Fragment durch den monoklonalen Antikörper 8G10/48, welcher durch die bei der Europäischen Sammlung von Tierzellkulturen, Porton Down, unter der Nr. 87081010 hinterlegte Zellinie gebildet wird, erkannt wird.

5. Verwendung eines Antigens oder antigenen Fragments, das nach einem der Ansprüche 1 bis 4 hergestellt wurde, zur Zubereitung eines Impfstoffs zur aktiven Immunisierung eines Wirts gegen Plasmodium falciparum.

6. Verfahren zur Herstellung eines Impfstoffs durch Vermischen eines Antigens oder antigenen Fragments, hergestellt nach einem der Ansprüche 1 bis 4, mit einem pharmazeutisch akzeptablen Träger oder Verdünnungsmittel.

7. Verfahren zur Herstellung eines Impfstoffs nach Anspruch 6, weiterhin umfassend das Zugeben eines Adjuvans.

8. Verfahren zur Herstellung eines Impfstoffs nach Anspruch 6 oder 7, wobei das Antigen die in Fig. 6 dargestellte Sequenz aufweist, oder ein antigenes Fragment desselben, welches in einem Wirt eine spezifische Immunantwort hervorruft, ist.

9. Verfahren zur Expression eines rekombinanten DNA-Moleküls, dadurch gekennzeichnet, daß das Molekül eine Nucleotidsequenz mit der Fähigkeit, als Polypeptid, welches eine Immunantwort auf ein Antigen oder ein antigenes Fragment, hergestellt nach einem der Ansprüche 1 bis 4, hervorzurufen vermag, exprimiert zu werden, umfaßt.

10. Verfahren zur Herstellung eines rekombinanten Klonierungsvehikels oder Vektors oder einer Wirtzelle durch Einführen des rekombinanten DNA-Moleküls gemäß der Definition in Anspruch 9 in ein Klonierungsvehikel, einen Vektor oder eine Wirtzelle.

11. Verfahren zur Herstellung eines synthetischen Polypeptids durch Exprimieren der gesamten oder eines Teils einer Nucleotidsequenz gemäß Definition in Anspruch 9.

12. Verfahren zur Herstellung eines Impfstoffs durch Vermischen eines synthetischen Polypeptids, hergestellt nach Anspruch 11, mit einem pharmazeutisch akzeptablen Träger.

## Revendications (Revendications pour l'(les) Etat(s) contractant(s) suivant(s): BE, CH, LI, DE, FR, GB, IT, LU, NL, SE)

1. Antigène isolé de surface de mérozoite des stades sanguins asexués de Plasmodium falciparum, n'appartenant pas aux isolats de P. falciparum FVO et Geneva, caractérisé en ce que :
(i) il a un poids moléculaire apparent dans une gamme d'approximativement 41 kDa à 53 kDa en PAGE SDS réducteur ;
(ii) c'est une glycoprotéine comprenant de l'acide myristique ;
(iii) il est d'abord présent sous forme diffuse dans le cytoplasme et chez les schizontes matures sur la membrane de surface des mérozoites ;
(iv) il est reconnu par des anticorps monoclonaux spécifiques des stades sanguins asexués de P. falciparum qui inhibent la croissance du parasite in vitro ; et
(v) lorsqu'il est isolé de la souche FCQ-27/PNG, il possède la séquence d'acides aminés indiquée à la figure 6 ;
et qui ne dérive pas du précurseur 195 kDa de l'antigène de surface mérozoite majeur (PMMSA) ;
ou fragment antigénique dudit antigène qui induit une réponse immunitaire spécifique chez un hôte.

2. Antigène isolé ou fragment antigénique selon la revendication 1, où l'antigène a la séquence d'acide aminé indiquée à la figure 6.

3. Antigène isolé ou fragment antigénique selon la revendication 1 ou 2, qui est caractérisé par l'inclusion dans sa séquence d'acides aminés de la (séquence Ser-Thr-Asn-Ser (STNS) ou la séquence Ser-Asn-Thr-Asn-Ser-Val (SNTNSV)).

4. Antigène isolé ou fragment antigénique de Plasmodium falciparum selon l'une quelconque des revendications 1 à 3, qui est reconnu par l'anticorps monoclonal 8G10/48 produit par la lignée cellulaire déposée auprès de la Collection Européenne de Culture de Cellules Animales (European Collection of Animal Cell Cultures, Porton Down), sous le n° 87081010.

5. Utilisation d'un antigène ou d'un fragment antigénique selon l'une quelconque des revendications 1 à 4, pour la préparation d'une composition vaccinale pour immuniser de façon active un hôte contre Plasmodium falciparum.

6. Composition vaccinale comprenant un antigène ou un fragment antigénique selon l'une quelconque des revendications 1 à 4, et un support ou un diluant pharmaceutiquement acceptable.

7. Composition selon la revendication 6, comprenant en outre un adjuvant.

8. Composition selon la revendication 6, où l'antigène a la séquence indiquée à la figure 6 ou un fragment antigénique de ce dernier qui induit une réponse immunitaire spécifique chez un hôte.

9. Molécule d'ADN recombinant comprenant une séquence nucléotidique capable d'être exprimée en un polypeptide capable d'induire une réponse immunitaire contre un antigène ou un fragment antigénique selon l'une quelconque des revendications 1 à 4, ou véhicule de clonage recombinant ou vecteur ou cellule hôte comprenant une telle molécule d'ADN recombinant.

10. Polypeptide synthétique préparé par l'expression de toute ou d'une portion de la séquence nucléotidique selon la revendication 9.

11. Composition vaccinale comprenant un polypeptide synthétique selon la revendication 10.

## Revendications (Revendications pour l'(les) Etat(s) contractant(s) suivant(s): AT)

1. Procédé pour la préparation d'un antigène isolé de surface de mérozoite des stades sanguins asexués de Plasmodium falciparum, n'appartenant pas aux isolats de P. falciparum FVO et Geneva, ledit antigène étant caractérisé en ce que :
(i) il a un poids moléculaire apparent dans une gamme d'approximativement 41 kDa à 53 kDa en PAGE SDS réducteur ;
(ii) c'est une glycoprotéine comprenant de l'acide myristique ;
(iii) il est d'abord présent sous forme diffuse dans le cytoplasme et chez les schizontes matures sur la membrane de surface des mérozoites ;
(iv) il est reconnu par des anticorps monoclonaux spécifiques des stades sanguins asexués de P. falciparum qui inhibent la croissance du parasite in vitro ; et
(v) lorsqu'il est isolé de la souche FCQ-27/PNG, il possède la séquence d'acides aminés indiquée à la figure 6 ;
et ne dérivant pas du précurseur 195 kDa de l'antigène de surface mérozoite majeur (PMMSA) ;
ou d'un fragment antigénique dudit antigène qui induit une réponse immunitaire spécifique chez un hôte,
ce procédé comprenant l'isolement dudit antigène ou fragment d'antigène à partir de cellules de P. falciparum ou de cellules recombinantes capables d'exprimer cet antigène.

2. Procédé selon la revendication 1, où l'antigène a la séquence d'acide aminé indiquée à la figure 6.

3. Procédé selon la revendication 1 ou 2, ledit antigène étant caractérisé par l'inclusion dans sa séquence d'acides aminés de la (séquence Ser-Thr-Asn-Ser (STNS) ou la séquence Ser-Asn-Thr-Asn-Ser-Val (SNTNSV)).

4. Procédé selon l'une quelconque des revendications 1 à 3, dans lequel ledit antigène ou fragment antigénique est reconnu par l'anticorps monoclonal 8G10/48 produit par la lignée cellulaire déposée auprès de la Collection Européenne de Culture de Cellules Animales (European Collection of Animal Cell Cultures, Porton Down), sous le n° 87081010.

5. Utilisation d'un antigène ou d'un fragment antigénique préparé selon l'une quelconque des revendications 1 à 4, pour la préparation d'une composition vaccinale pour immuniser de façon active un hôte contre Plasmodium falciparum.

6. Procédé pour la préparation d'une composition vaccinale, comprenant le mélange d'un antigène ou d'un fragment antigénique selon l'une quelconque des revendications 1 à 4, avec un support ou un diluant pharmaceutiquement acceptable.

7. Procédé pour la préparation d'une composition vaccinale selon la revendication 6, comprenant en outre l'addition d'un adjuvant.

8. Procédé pour la préparation d'une composition vaccinale selon la revendication 6 ou la revendication 7, où ledit antigène a la séquence indiquée à la figure 6 ou un fragment antigénique de ce dernier qui induit une réponse immunitaire spécifique chez un hôte.

9. Procédé pour l'expression d'une molécule d'ADN recombinant, caractérisé en ce que ladite molécule comprend une séquence nucléotidique capable d'être exprimée en un polypeptide capable d'induire une réponse immunitaire contre un antigène ou un fragment antigénique selon l'une quelconque des revendications 1 à 4.

10. Procédé pour la préparation d'un véhicule de clonage recombinant ou vecteur ou cellule hôte comprenant l'introduction d'une molécule d'ADN recombinant, telle que définie dans la revendication 9, dans un véhicule de clonage ou un vecteur ou une cellule hôte.

11. Procédé pour la préparation d'un polypeptide synthétique comprenant l'expression de toute ou d'une portion de la séquence nucléotidique selon la revendication 9.

12. Procédé pour la préparation d'une composition vaccinale comprenant le mélange d'un polypeptide synthétique selon la revendication 11 avec un diluant pharmaceutiquement acceptable.
